# EUROPEAN PATENT APPLICATION

(11) **EP 4 249 024 A1**
(43) Date of publication of application: **27.09.2023**
(21) Application number: 22163565.9
(22) Date of filing: 22.03.2022
(51) Int. Cl.: A61M 16/06

(54) **MINIMUM CONTACT RESPIRATORY NASAL MASK**

(71) Applicant: Air Liquide Medical Systems, 92182 Antony Cedex (FR)
(72) Inventor: ALBERICI, Luca, 25073 Bovezzo (IT); RUOCCO, Alessandra, 25073 Bovezzo (IT); SANDONI, Giuseppe, 25073 Bovezzo (IT)
(74) Representative: Air Liquide

(57) **Abstract**

A respiratory nasal mask (1) comprising a mask body (10) comprising a central opening (12) and two lateral arms (13) projecting laterally on each side of said mask body (10), a flexible cushion (30) and a rigid ring (20) sandwiched in-between. The mask body (10) comprises an oval-shape structure (11) with a flat curved front surface (10a). The peripheral edge regions (34) surrounding the rear breathing opening (33) of the flexible cushion (30) are configured to, in use, come into contact and provide a gas sealing with areas located immediately around the nostril edges of the user comprising an upper lip area (ULA) located immediately under the nostrils, two curved alae (CA) on both sides of the nostrils and a tip region (TR) of the nose of the user, thereby minimizing the contact areas with the user's face. The mask (1) of the present invention is useable for treatment of a respiratory disorders or conditions, such as OSA.

## Description

The invention concerns a respiratory nasal mask comprising a flat curved body, a central ring and a flexible nasal cushion coupled or attached together, that is useable in the treatment of respiratory conditions or diseases affecting adults, children, toddlers, infants or newborns, such as obstructive sleep apnea (OSA).

Respiratory masks are commonly used for delivering a flow of breathable gas, such as air under pressure, for, or to assist in, patient respiration.

A mask assembly typically comprises a rigid or semi-rigid hollow shell or body, usually made of polymer, and further comprising a soft face-contacting cushion, commonly called a flexible cushion, that comes into contact with the patient's face and conforms to the various facial contours of the patient face for receiving the patient's nose and/or mouth so that the patient can inhale the gas comprised into a breathing chamber that is defined by the hollow shell or body and/or the flexible cushion.

The flexible cushion is usually made of soft, resilient elastomeric material, such as soft silicone or similar material.

A gas supply line delivers a respiratory gas, such as air under pressure, to the mask, namely the breathing chamber. An example of such a mask is given by EP-A-462701.

A headgear, comprising flexible straps or similar structures, is usually attached to the mask for correctly positioning, holding and/or securing the mask on the head of a patient. The headgear can be adjusted to pull the mask against the face with sufficient force to achieve a gas tight seal between the mask and the wearer's face as taught by EP-A-462701, EP-A-874667, EP-A-1972357 or WO-A-00/57942.

Other types of masks comprising a front shroud adapted to attach the headgear are disclosed by EP-A-2708258 or US-A-2007/0044804.

Masks receiving only the patient's nose are called a nasal mask, whereas those receiving the patient's nose and mouth are called facial or oro-nasal masks. Such masks are commonly used for treating respiratory conditions or diseases affecting adults or children, including some toddlers, infants or newborns, such as obstructive sleep apnea (OSA). For instance, the treatment of OSA or the like, for being efficient, requires that the patient uses the nasal mask overnight, namely when he/she sleeps.

Despite the fact that a lot of different architectures or masks have been proposed in the prior, especially for treating OSA or the like, problems or drawbacks still exist with existing nasal masks.

Thus, one important problem is related to the discomfort of use for the patient. Various causes of discomfort exist, such as gas leaks due to a wrong assembling of the mask components, a poor positioning of the mask of the patient's face, loose settings of the headgear, poor tightness of the cushion...

Gas leaks due to wrong assembling of the mask components can arise when the complexity of the mask is excessive, knowing that a patient has to disassemble and then re-assemble the components of the mask on regular basis, for instance every morning for cleaning them up.

Further, gas leaks due to a poor positioning of the mask can appear when the mask is too voluminous and hence difficult to be well-positioned by a user on his/her face, and/or to insufficient tightness of the cushion due to a poor contacting between the mask and patient's facial areas, especially for too heavy masks, that can be aggravated by loose settings of the headgear.

One can easily understand that those gas leaks and related discomfort for the patient can lead to a poor or inefficient treatment, including to an abandonment of their treatment by some patients.

In this context, a goal of the present invention is to provide an improved nasal mask having a straightforward architecture for the patients and leading to an efficient tightness, thereby avoiding or minimizing the risks of incorrect assembling of different components, gas leaks and related discomfort for the patient.

In other words, the goal of the present invention is to provide an improved respiratory nasal mask architecture that is compact, simple to assemble, light and comfortable to wear, thanks to an improved architecture, thereby ensuring efficient positioning and securing of the mask on the patient's face, good comfort of use for the patients and an efficient gas tightness, i.e. seal, preventing the escape of gas that may result from a poor coupling of the components of the mask and/or from a poor contacting between the cushion and the facial areas of the patient.

A solution of the present invention concerns a respiratory nasal mask comprising:
- a mask body comprising a central opening and two lateral arms projecting laterally on each side of said mask body,
- a flexible cushion defining an inner breathing chamber comprising a front gas orifice in fluid communication with the central opening of the mask body, and a rear breathing opening for allowing, in use, gas exchanges with the nostrils of a user, and
- a rigid ring traversed by an inner passage, sandwiched between the mask body and the flexible nasal cushion.

Furtermore, according to the invention :
- the mask body comprises an oval-shape structure comprising a flat curved front surface and a rear surface, and traversed by the central opening,
- the oval-shape structure and the two lateral arms of the mask body are made in one piece,
- the flexible nasal cushion is attached to the rear surface of the oval-shape structure and
- the peripheral edge regions surrounding, i.e. located around, the rear breathing opening of the flexible nasal cushion are configured to, in use, come into contact and provide a gas sealing with areas located immediately around the nostril edges of the user comprising an upper lip area (ULA) located immediately under the nostrils, two curved alae (CA) on both sides of the nostrils, and a tip region (TR) of the nose.

The respiratory nasal mask according to the present invention can further comprise one or more of the following features.

### Mask body

The mask body of the nasal mask according to the present invention can further comprise one or more of the following additional features:
- it comprises a central opening having a circular shape.
- the central opening has a diameter of between 15 and 25 mm, preferably of between 17 and 22 mm.
- it is made of polymer, i.e. a plastic material, such as polycarbonate (PC), polypropylene (PP), nylon, or the like, for instance PC.
- it is molded in one piece with the two lateral arms.
- the two lateral arms project laterally on the left and right sides of the mask body, namely in opposite directions.
- the two lateral arms have an elongated shape, such as a band or ribbon shape. Other shapes are of course also possible.
- the lateral arms have each a length of about 3 to 10 cm, preferably of about 3 to 6 cm.
- each lateral arm comprise a free end with headgear connecting structures.
- the headgear connecting structures comprise one or several slots, hooks or the like.
   a headgear is attached to the headgear connecting structures of each lateral arm. Preferably, the headgear comprises flexible straps or the like for correctly positioning, holding and/or securing the mask on the head of a patient.
- the oval-shape structure has a length (L) of between 40 and 70 mm and a width (W) of between 30 and 50 mm.
- the oval-shape structure has a thickness of between 2 and 5 mm.
- the oval-shape structure comprises a curved front surface located toward the outside.
- the oval-shape structure forms or comprises a curved wall or the like.
- the curved front surface of the oval-shape structure is flat.
- the oval-shape structure is slightly foldable, i.e. can be slightly bent, especially when tension forces are applied by the headgear connected to the two arms.
- the oval-shape structure comprises a rear surface facing the cushion.
- the oval-shape structure comprises two opposite tips, the two lateral arms being attached to said two opposite tips.
- the central opening is arranged at the center of the oval-shape structure.
- the mask body comprises a tubular structure comprising the central opening traversing the mask body, i.e. the central opening traversing the mask body is in fluid communication with the lumen of a tubular structure attached to the mask body, namely to the rear surface facing the cushion.
- the central opening constitutes an inlet of the tubular structure.
- the tubular structure is made one piece with the mask body, in particular with the oval-shape structure.
- the tubular structure forms a collar projecting away from the rear surface of the mask body, i.e. toward the cushion, especially the rear surface of the oval-shape structure.
- the rear surface of the mask body comprises connecting means for attaching the rigid ring thereto.
- the connecting means are arranged around and spaced from the tubular structure.
- the connecting means comprise wall elements or the like.
- the mask body further comprises several venting orifices or holes are arranged around the central opening of the mask body.
- the venting orifices traverse the mask body, i.e. traversing holes, and are in fluid communication with the inner breathing chamber of the flexible nasal cushion.
- the venting orifices have a cylindrical or tronconical shape.
- the venting orifices are arranged in groups or arrays comprising each several venting orifices.
- groups or arrays of venting orifices are arranged so as to sandwich the central hole, i.e. arranged to the left and to right of the central hole.
- the venting orifices traversing the mask body lead, on the rear surface, between the tubular structure and the connecting means for attaching the rigid ring.
- the central opening of the mask body, the inner passage of the rigid ring and the front gas orifice of the flexible nasal cushion are coaxially-arranged (AA).

### Cushion

The cushion of the nasal mask according to the present invention can further comprise one or more of the following additional features:
- it is sized and designed for not covering the regions of the nose of the user situated above his/her nose tip or tip region (TR), when the mask is worn by said user, especially the dorsum, i.e. cartilaginous and/or bony dorsum.
- it is made of a flexible material, such as silicone or the like, i.e. a soft or semi-soft material.
- it comprises an annular portion surrounding the gas orifice.
- the inner breathing chamber of the cushion comprises a rear opening adapted for providing gas to the patient's nostrils.
- the rear breathing chamber has (almost or quasi) rectangular or trapezoidal shape.
- the peripheral edge regions externally surround the rear breathing opening of the flexible nasal cushion.
- the peripheral edge regions surrounding the rear breathing opening of the flexible nasal cushion comprise a peripheral thin wall, i.e. a peripheral flexible membrane or the like, forming a sealing flexible skirt around said rear breathing opening so as to ensure an efficient gas tightness while being in contact with the patient's face, when the latter wears the mask, preferably all along the outer periphery of the aperture of the respiratory chamber.
- the peripheral thin wall forming the sealing flexible skirt surrounding the rear breathing opening comes into contact and provide a gas sealing with the areas located immediately around the nostril edges of the use, namely the upper lip area (ULA), the two curved alae (CA), and the tip region (TR) of the nose.
- the rear surface of the flexible nasal cushion form is curved, in particular for matching, in use, the two curved alae (CA) of the nose of the user.
- the peripheral edge regions surrounding the rear breathing opening of the flexible nasal cushion form a curved (quasi) contacting surface, preferably a triangular or trapezoidal curved contacting surface.
- the curved (quasi) contacting surface surrounding the rear breathing opening comes into contact, in use, i.e. when the user wears the mask, with the areas (i.e. user's facial areas) located immediately around the nostril edges of the user thereby providing an efficient gas sealing.
- the rear breathing opening having a (almost or quasi) rectangular or trapezoidal shape is arranged in the curved (quasi) triangular contacting surface located around the rear breathing opening, preferably in the center of said curved (quasi) triangular contacting surface.
- the peripheral edge regions of the rear breathing opening of the flexible nasal cushion comprises a base border, two lateral panels or wings, and a top border.
- the width W1 of the top border is larger/greater than the width W2 of the base border, i.e. W1>W2.
- the width W1 is of between 17 and 25 mm.
- the width W2 is of between 10 and 20 mm.
- the inner breathing chamber is in fluid communication with the gas passage of a hollow gas connector providing gas, such as air, to the central opening of the mask.
- the rear aperture is in fluid communication with the inner chamber of the cushion.
- the peripheral thin wall forming the sealing skirt around the rear breathing opening is molded in one piece with the rest of the cushion.

### Ring

The ring of the nasal mask according to the present invention can further comprise one or more of the following additional features:
- it is attached to the flexible cushion.
- it is non-removably attached to the flexible cushion, i.e. the ring cannot be easily detached and is not supposed to be detachable from the flexible cushion by a user.
- alternatively, it is removably attached to the flexible cushion, i.e. it can be detached, for instance for cleaning it up.
- it is non-removably snap-fitted or similar to the flexible cushion.
- it has a generally annular shape, i.e. a ring shape.
- it is made of plastic material, such as polybutylene terephthalate (PBT), polyamide (PA), polycarbonate (PC) and polypropylene (PP), or the like, preferably PBT.
- it is a rigid ring.
- the inner passage of the ring has a diameter of between 1 and 4 cm.

### Connecting structure

The mask body, the ring and the cushion of the nasal mask according to the present invention comprise connecting structures for attaching the ring to the cushion, and the cushion to the rear face of the mask body, namely to the oval-shape structure.

Said connecting structures can comprise walls, abutments, lodgings and grooves, or the like, forming snap-fit connecting structures, threaded connecting structures, bayonet connecting structures, plug-in structures or any other suitable connecting structures.

### Hollow gas connector

The gas connector of the nasal mask according to the present invention can further comprise one or more of the following additional features:
- it is a straight connector, i.e. tubular shape.
- it is fixed to the mask body, i.e. held in position on the mask body.
- it is detachably fixed to the mask body.
- it is rotatable with respect to the mask body.
- it is configured for connecting a gas line thereto, i.e. a flexible hose or the like. In other words, the tubular connector is adapted for receiving a flexible gas conduct or hose that feeds a respiratory gas under pressure to the respiratory mask. The gas is fed into the flexible gas conduct or hose by a gas source, such as a medical ventilator, a medical gas-delivery device or the like.
- it is in fluid communication with the breathing chamber of the cushion thereby allowing gas to circulate from the hollow connector to the cushion.
- it comprises a proximal end that is inserted and held in the central opening of the mask body, when the hollow connector is attached to the mask body.
- the proximal end of the hollow connector has a cylindrical or tronconical shape.
- according to another embodiment, the connector maybe a hollow curved or elbow connector, i.e. having a "L-shape" or the like.
- it is made of polymer material, e.g. plastic.

### Headgear

The headgear of the nasal mask according to the present invention can further comprise one or more of the following additional features:
- it is attached to the headgear connection structures of the two lateral arms.
- it comprises straps that are used for maintaining and securing the mask in a desired position on the facial regions of the patient.
- the straps are made of polymer material or fabric material, or both.
- the straps comprise or are elongated flexible bands, ribbons or the like.
- the straps constitute a tridimensional structure.

More generally, the invention also concerns a respiratory assembly for providing gas to a patient (P) comprising a gas delivery device, such as a medical ventilator/device or the like, and a nasal mask according to the present invention, wherein the gas delivery device is connected to the nasal mask by means of a gas line, such as a flexible hose.

Typically, the gas line is connected to the tubular connecting connector fixed to the hollow gas connector, said hollow gas connector being itself fixed to the mask body.

Such a respiratory assembly using a nasal mask according to the present invention is useable in the treatment of respiratory conditions or diseases affecting adults, children, toddlers, infants or newborns, such as obstructive sleep apnea (OSA).

A non-limitative embodiment of a respiratory nasal mask according to the present invention is shown in the enclosed Figures, among which:
- Figure 1 represents an exploded view of a nasal mask according to one embodiment of the present invention,
- Figure 2 is a 3/4 view of the mask body of the nasal mask of Figure 1,
- Figure 3 is front view of the mask body of Figure 2,
- Figures 4 is a top view of the mask body of Figures 2 and 3,
- Figures 5 and 6 shows the nasal mask of Figure 1, worn by a patient,
- Figure 7 is a front view of a second embodiment of the mask body of a nasal mask according to the invention,
- Figure 8 is a top view of Figure 7 showing the mask body and the cushion attached thereto,
- Figure 9 is a lateral view of Figure 8 showing the mask body and the cushion attached thereto,
- Figure 10 is a rear view of Figures 8 and 9, showing the cushion.
- Figure 11 is a top view of Figure 10,
- Figure 12 is a top view showing the mask body and the cushion, worn by a patient,
- Figure 13 is a rear view of the mask body of Figure 7 and the rigid ring attached thereto, and
- Figures 14 and 15 are cross-sectional views of the mask of Figures 5-13.

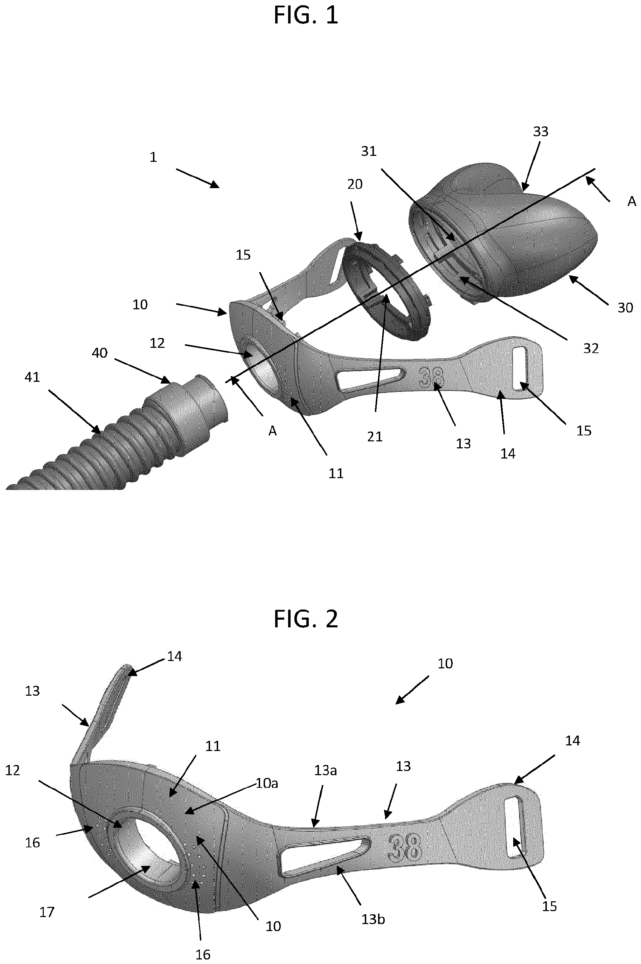

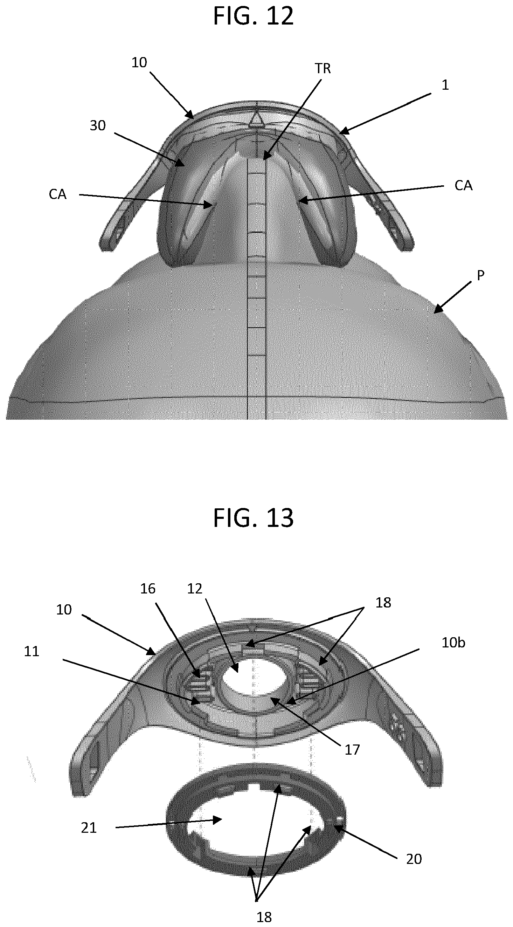

The present invention proposes a new structure of a respiratory nasal mask as shown in Figures 1-15 that illustrate embodiments of a nasal mask according to the present invention.

Generally speaking, a nasal mask 1 according to the present invention comprises three main parts assembled together, namely a mask body 10, a flexible cushion 30 and a rigid ring 20 sandwiched between said mask body 10 and flexible cushion 30.

The ring 20 is preferably firmly attached to the cushion 30 and normally not removable, whereas the cushion 30 equipped with the ring 20 is removably attached to the rear surface or rear side 10b of the mask body 10.

This mask 1 has a simple and light structure, and further facilitates the assembling/disassembling of the different components of the mask 1 by the users, namely the patients, especially during daily cleaning operations. Such a mask 1 further exhibits a good comfort as it is lighter and has a low risk of gas leaks as explained below.

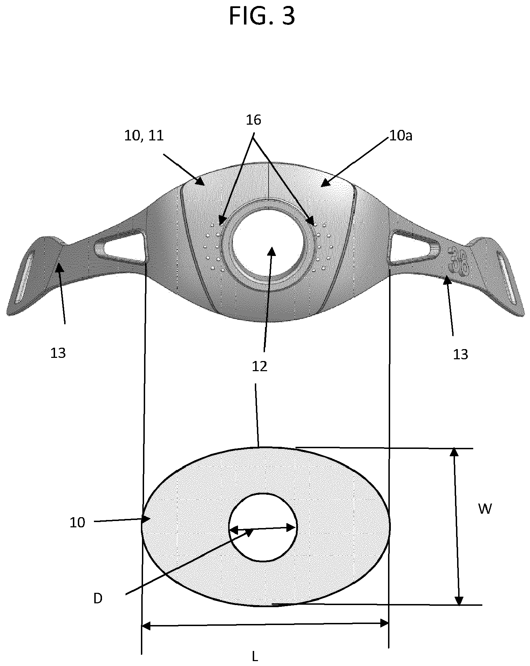

As shown in the Figures, the nasal mask 1 of the present invention comprises a mask body 10 comprising two lateral arms 13 projecting laterally, in opposite directions, i.e. one toward the right side of the mask 1 and the other toward the left side of the mask 1 as shown in Figure 3 for instance. The two lateral arms 13 are arranged around a central opening 12 traversing the mask body 10.

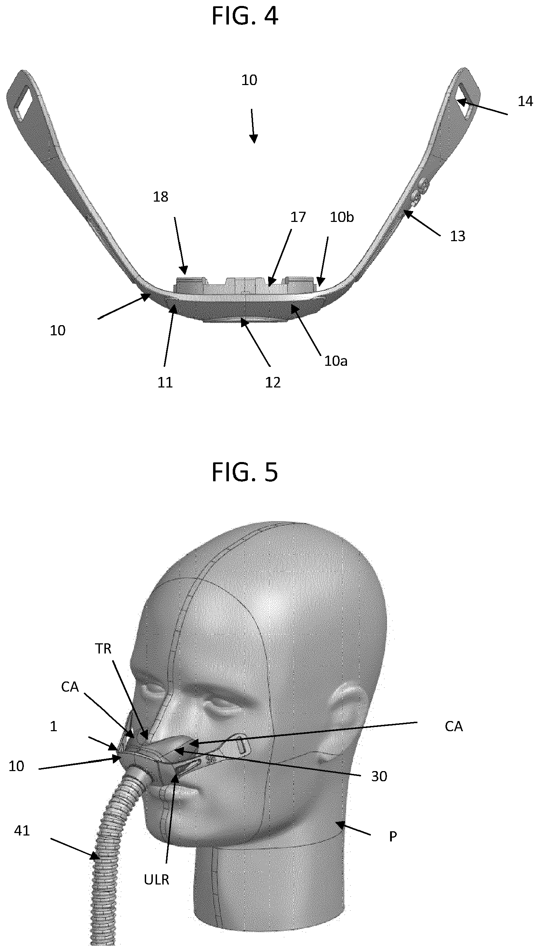

The two lateral arms 13 that project laterally may also constitute right and left cheek supports, when the mask is worn by a patient, as shown in Fig. 5-6.

As shown in the Figures, each lateral arm 13 has a length of about 3 to 10 cm, preferably of about 3 to 6 cm.

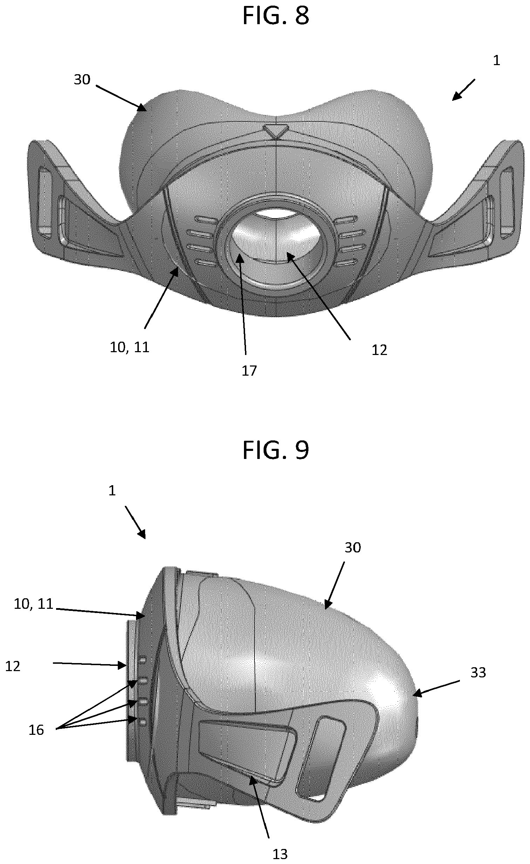

In a particular embodiment, the lateral arms 13 can be partially divided into two parallel spaced sub-arms as represented in Fig. 2, for thereby improving its flexibility and lowering its weight. However, this is not mandatory and the lateral arms 13 can also be solid, i.e. not divided, as shown in Fig. 8-13.

Preferably, the two lateral arms 13 have elongated shapes, such as band or ribbon shapes, and can be slightly incurved to better match, if desired, some of the contours of the cushion 30.

Further, the lateral arms 13 comprise a free end 14 equipped with headgear connecting structures 15, such one or several slots, hooks or the like. These headgear connecting structures 15 are designed for attaching a headgear thereto (not shown), typically the flexible straps of a headgear. A headgear (not shown) is commonly used for maintaining and securing the mask 1 in a desired position on the head of the patient, as explained below. Preferably, the flexible straps of the headgear are made of polymer or fabric materials.

According to the present invention, the mask body 10 comprises an oval-shape structure 11, as shown in Figure 3, comprising a flat curved front surface 10a and a rear surface 10b. Thanks to said oval shape 11, the mask body 10 can be light and easy to put in place by the user and further such a particular structure simplifies the coupling with the assembly cushion/ring 30,20 in avoiding misalignments or the like.

In the frame of the present invention, "oval shape" means a shape that is oval or approximatively oval, such as ellipsoidal or the like, as illustrated in Fig. 3.

The oval-shape structure 11 of the mask body 10 is traversed by the central opening 12 that can have an inner diameter of about between 1 and 4 cm.

The mask body 10, namely the oval-shape structure 11 and the two arms 13, are made of plastic material and molded in one piece, for instance by injection molding or any other suitable molding process.

Further, the central opening 12 of the mask body 10 is arranged at the center of the oval-shape structure 11 of the mask body 10, and comprises an inner diameter of between 15 and 25 mm.

The mask body 10 further comprises a tubular structure 17, as shown in Fig. 4, 8, 14 and 15, comprising the central opening 12 traversing the mask body. In other words, the central opening 12 of the mask body 10 is in fluid communication with the lumen of a tubular structure 17 attached to the rear surface 10b of the oval-shape structure 11 of the mask body 10. The central opening 12 constitutes an inlet of the tubular structure 17. Preferably, the tubular structure 17 is made one piece with the mask body 10, in particular with the rear surface 10b of the oval-shape structure 11. The tubular structure 17 forms a collar projecting away from the rear surface 10b of the oval-shape structure 11 of the mask body 10, i.e. toward the cushion 30. The collar of the tubular structure 17 cooperates with the rigid ring 20 for keeping the cushion 3 connected to the mask body 10 forming the main frame of the mask.

Furthermore, the rigid ring 20, that is made of a plastic or polymer material, is traversed by an inner passage 21 and is sandwiched between the mask body 10 and the flexible cushion 30, preferably the rigid ring 20 is non-removably attached to the flexible cushion 30.

Preferably, the rigid ring 20 is also made of plastic material. The main role of the ring 20 is to facilitate the quick coupling by a user of the cushion 30 to the rear surface or side 10b of the oval-shape structure 11 of the mask body 10, for example for daily cleaning operations or the like.

Typically, according to the present invention, the plastic or polymer material that is used for manufacturing the mask body 10 and/or the ring 20 can be PP, PC, PBT or nylon, or similar materials.

The nasal mask 1 of the invention further comprises a flexible cushion 30, preferably made of silicone, namely a tridimensional hollow flexible structure defining a breathing chamber 31, and further comprising a rear breathing opening 33 and a front gas orifice 32, both in fluid communication with the breathing chamber 31.

The flexible cushion 30 also comprises a contact zone located on its outer surface that comes into contact, in use, with the patient's face, namely regions located immediately around the nostrils of the patient P as below explained.

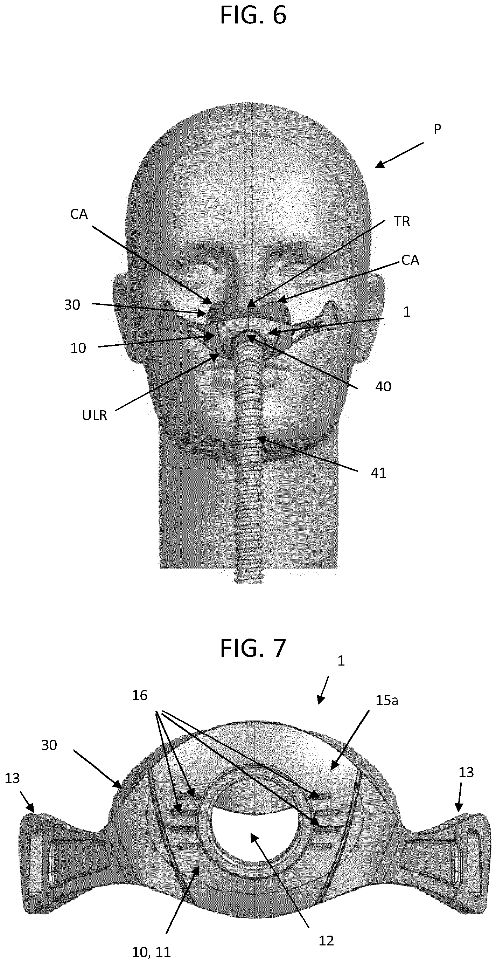

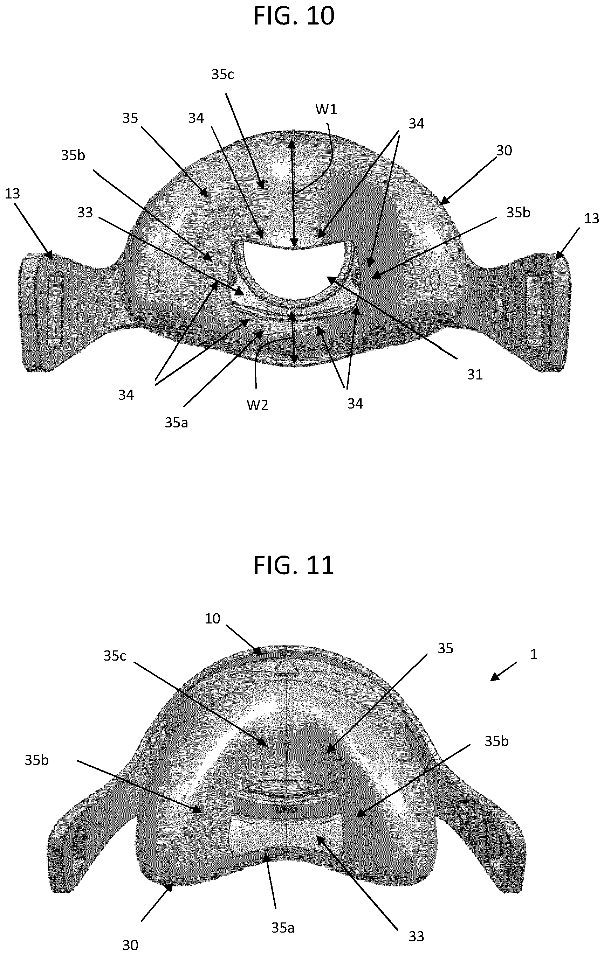

More precisely, said contact zone comprises the peripheral edge regions 34 located, on its outer surface, immediately around the rear breathing opening 33 of the flexible cushion 30, as shown in Figures 10 & 11. Said peripheral edge regions 34 are configured to, in use, come into contact and provide a gas sealing with areas located immediately around the nostril edges of the user, i.e. a patient P, as shown in Fig. 5 & 6, comprising :
. an upper lip area (ULA) located immediately under the nostrils of the user P,
. two curved alae (CA) on both sides of the nostrils of the user P, and
. a tip region (TR) of the nose of the user P.

In other words, the cushion 30 of the nasal mask 1 of the present invention has a compact size and does not extend over the top of the nose, namely does not cover the dorsum regions, i.e. cartilaginous and/or bony dorsum, when the mask 1 is worn by a user P as shown in Fig. 5, 6 & 12.

Such a compact cushion 30 provides several advantages such as it can easily adapted to more patients having various facial morphologies, since the prominence of their nose has no effect, when compared to traditional cushions where the top of the patient's nose has to be inserted far into the breathing chamber and hence can touch some internal parts.

Generally speaking, the compact cushion 30 of the nasal mask 1 of the present invention reduces and/or minimizes the contact with the skin of the user as it rests only on the areas located immediately around the entrances of the nostrils of said user. In other words, the compact cushion 30 of the invention can be sized and/or designed in taking into account only the width of the bottom of the nose of the user.

Furthermore, in order to provide efficient gas tightness (i.e. seal) and/or good comfort for the patient, the cushion wall in the peripheral edge regions 34 surrounding the rear opening 33 of the cushion 30 can be conceived as a flexible membrane that comes into contact with the patient's facial areas or regions, namely the upper lip area (ULA), the two curved alae (CA) and tip region (TR) as above stated, so as to better match his/her facial morphology. Said flexible membrane forms a soft skirt or the like all along the edges of the rear opening 33 of the cushion 30. A unique membrane can be used or, in alternative embodiments, several membranes may be superimposed.

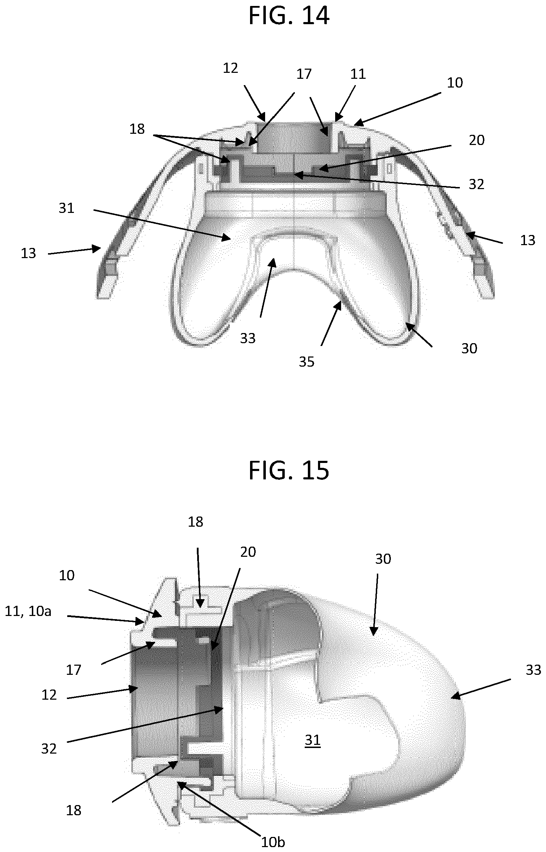

As better shown in Fig. 11 & 14, the rear surface 35 of the cushion 30 has a curved shape for better matching some nasal regions, in particular the two curved alae (CA) situated on both sides of the nostrils of the user P.

Further, as shown in Fig. 10, the rear breathing opening 33 has a (quasi-) rectangular or similar (e.g. trapezoidal shape). It is arranged at the center of the curved rear surface 35 that has also a curved (quasi) triangular or trapezoidal contacting surface including the peripheral edge regions 34 surrounding the rear breathing opening 33 of the flexible nasal cushion 30.

More precisely, as shown in Fig. 10, the outer curved rear surface 35 of the peripheral edge regions 34 surrounding the rear breathing opening 33 of the flexible nasal cushion 30 comprises :
- a base border 35a that comes, in use, into contact with the upper lip area (ULA) of the user,
- two lateral panels or wings 35b that come, in use, into contact with the two curved alae (CA) on both sides of the nostrils of the user P, and
- a top border 35c that comes, in use, into contact with the tip region (TR) of the nose of the user P.

The width W1 of the top border 35c is greater than the width W2 of the base border 35a. For instance, W1 is comprised between 17 and 25 mm, whereas W2 is comprised between 10 and 20 mm.

In other words, the cushion 30 has a tridimensional form or shape (from a rear view) that well-matches the contours of the patient's nasal regions (i.e. ULA, CA, TR), including a curved rear surface 25 having a trapezoidal or triangular shape or similar, thereby forming a contact zone constituting a gas seal (i.e. improved tightness), as illustrated in Figure 10, showing the rear surface 35 of the cushion 30 comprising the rear opening 33 and the surrounding peripheral edge regions 34.

The cushion 30 is directly coupled to the ring 20, whereas the ring 20 is itself coupled to the rear surface 10b of the mask body 10, namely the oval-shape structure 11 attached to the two lateral arms 13.

Preferably, cushion 30, ring 20 and mask body 10 can be detached for cleaning operations or the like. However, in another embodiment, the ring 20 cannot be detached from the cushion 30.The rigid ring 20 is itself firmly held in place in the body of the flexible cushion 3 by a tight connection system, such as snap-fitting, threading, a bayonet system or any other connection system, including over-molding.

Further, the front orifice 32 of the flexible cushion 30 is coaxially arranged and in fluid communication with the central opening 12 of the mask body 10 and the inner passage 21 of the rigid ring 20 so that the gas can travel through said central opening 12, inner passage 21 and front orifice 32.

A respiratory gas, such as air, provided by a flexible hose 41 fluidly and mechanically connected to the mask body 10 by a gas connector 40, can enter into the breathing chamber 31 of the cushion 30 passing through the front gas orifice 32, whereas gases expired by the patient, that are enriched in CO₂, can exit of the breathing chamber 31 by a plurality of venting orifices 16 in fluid communication with the inner breathing chamber 31 and afterwards can be vented to the atmosphere as said venting orifices 16 fluidly communicate with the atmosphere.

More precisely, the venting orifices 16 are located around the central opening 12, some instance on the right and left sides of it, and traverse the wall of the oval-shape structure 11 as shown in Fig. 2-3 & 7-8.

The venting holes 51 have preferably a tronconical-shape passage for the gas with an inner diameter greater than an outer diameter.

Further, the venting orifices 16 traversing the mask body 10 lead, on the rear surface 10b of the oval-shape structure 11, between a tubular structure 17 and connecting means 18 configured for attaching the rigid ring 20.

Furthermore, for ensuring a firm attachment of the cushion 30 to the ring 20, and to the mask body 10, it is provided suitable connecting means 18 or structures, such as walls, abutments, lodgings and grooves, or the like, cooperating together, for instance forming snap-fit connecting structures, threaded connecting structures, bayonet connecting structures, plug-in structures or any other suitable connecting structures.

Generally speaking, the compact nasal mask 1 of the present invention is light and comfortable to wear.

Such a simple architecture leads to a reduction of the overall size and weight of the mask 1, allowing easy assembling/disassembling operations and efficient positioning and securing of the mask 1 on the patient's nasal regions, as well as a good tightness (i.e. seal) and an improved comfort of use for the patient.

Generally speaking, the nasal mask 1 of the present invention can be used in a method for treatment of a respiratory disorder or condition affecting infant, toddler, child or adult patients, such as OSA.

## Claims

1. Respiratory nasal mask (1) comprising:
- a mask body (10) comprising a central opening (12) and two lateral arms (13) projecting laterally on each side of said mask body (10),
- a flexible cushion (30) defining an inner breathing chamber (31) comprising a front gas orifice (32) in fluid communication with the central opening (12) of the mask body (10), and a rear breathing opening (33) for allowing, in use, gas exchanges with the nostrils of a user (P), and
- a rigid ring (20) traversed by an inner passage (21), sandwiched between the mask body (10) and the flexible nasal cushion (30),
**characterized in that** :
- the mask body (10) comprises an oval-shape structure (11) comprising a flat curved front surface (10a) and a rear surface (10b), and traversed by the central opening (12),
- the oval-shape structure (11) and the two lateral arms (13) of the mask body (10) are made in one piece,
- the flexible nasal cushion (30) is attached to the rear surface (10b) of the oval-shape structure (11) and
- the peripheral edge regions (34) surrounding the rear breathing opening (33) of the flexible cushion (30) are configured to, in use, come into contact and provide a gas sealing with areas located immediately around the nostril edges of the user comprising an upper lip area (ULA) located immediately under the nostrils, two curved alae (CA) on both sides of the nostrils and a tip region (TR) of the nose.

2. Mask according to Claim 1, **characterized in that** the mask body (10) and the two lateral arms (13) are made in one piece of polymer material, preferably molded in one piece, and the flexible nasal cushion (30) is made of a flexible material, preferably silicone.

3. Mask according to anyone of Claims 1 or 2, **characterized in that** the mask body (10) comprises a tubular structure (17) comprising the central opening (12) traversing the mask body (10), said tubular structure (17) forming a collar projecting away from the rear surface (10b) of the mask body (10).

4. Mask according to Claims 1 and 2, **characterized in that** the rear surface (10b) of the mask body (10) comprises connecting means (18) for attaching the rigid ring (20) thereto, said connecting means (18) being arranged around and spaced from the tubular structure (17).

5. Mask according to Claim 1, **characterized in that** the mask body (10) further comprises several venting orifices (16) are surrounding the central opening (12) of the mask body (10), said venting orifices (16) traversing the mask body (10) and being in fluid communication with the inner breathing chamber (31) of the flexible nasal cushion (30).

6. Mask according to Claims 4 and 5, **characterized in that** the venting orifices (16) traversing the mask body (10) lead, on the rear surface (10b), between the tubular structure (17) and the connecting means (18) for attaching the rigid ring (20).

7. Mask according to Claim 1, **characterized in that** the inner passage (21) of the rigid ring (20), the front gas orifice (32) of the flexible nasal cushion (30) and the central opening (12) of the mask body (10) are coaxially-arranged (AA).

8. Mask according to any one of the preceding Claims, **characterized in that** the rigid ring (4) is made of polymer material.

9. Mask according to any one of the preceding Claims, **characterized in that** the outer rear surface (35) comprising the peripheral edge regions (34) surrounding the rear breathing opening (33) of the flexible nasal cushion (30) has a curved surface.

10. Mask according to Claim 9, **characterized in that** the outer curved rear surface (35) of the peripheral edge regions (34) surrounding the rear breathing opening (33) of the flexible nasal cushion (30) comprises :
- a base border (35a) that comes, in use, into contact with the upper lip area (ULA) of the user (P),
- two lateral panels or wings (35b) that come, in use, into contact with the two curved alae (CA) on both sides of the nostrils of the user (P), and
- a top border (35c) that comes, in use, into contact with the tip region (TR) of the nose of the user (P).

11. Mask according to any one of the preceding Claims, **characterized in that** the outer rear surface (35) of the flexible nasal cushion (30) has a trapezoidal or triangular shape and/or rear breathing opening (33) of the flexible nasal cushion (30) has a rectangular or trapezoidal shape

12. Mask according to any one of the preceding Claims, **characterized in that** the the wall of the flexible nasal cushion (30) comprise a peripheral thin wall surrounding the rear breathing opening (33) of the flexible nasal cushion (30) forming a sealing flexible skirt around said rear breathing opening (33), said flexible skirt comprising the base border (35a), the two lateral panels (35b) and the top border (35c).

13. Mask according to any one of the preceding Claims, **characterized in that** the peripheral edge regions of the rear breathing opening (33) of the flexible nasal cushion (30) comprises a base border, two lateral panels or wings, and a top border, the width (W1) of the top border being larger/greater than the width (W2) of the base border.

14. Mask according to any one of the preceding Claims, **characterized in that** it further comprises a headgear attached to headgear connection structures (15) of the two lateral arms (13).

15. Respiratory assembly for providing gas to a patient (P) comprising a gas delivery device and a respiratory nasal mask (1) according to any one of the preceding Claims, said gas delivery device being fluidly and mechanically connected to the respiratory nasal mask (1) by means of a gas line (41) equipped with a gas connector (40) plugged into the central opening (12) of the mask body (10).
